# EUROPEAN PATENT APPLICATION

(11) **EP 4 350 417 A1**
(43) Date of publication of application: **10.04.2024**
(21) Application number: 22837876.6
(22) Date of filing: 28.06.2022
(51) Int. Cl.: G02B 27/01, G02B 27/00, G06T 19/00, A61B 3/032, A61B 3/00

(54) **AUGMENTED REALITY APPARATUS AND METHOD FOR PROVIDING VISION MEASUREMENT AND VISION CORRECTION**

(30) Priority: 05.07.2021 KR 20210088079
(71) Applicant: Samsung Electronics Co., Ltd., Suwon-si, Gyeonggi-do 16677 (KR)
(72) Inventor: LEE, Kyookeun, Suwon-si, Gyeonggi-do 16677 (KR); MILTON, Harry Edward, Suwon-si, Gyeonggi-do 16677 (KR); SHIN, Sunghwan, Suwon-si, Gyeonggi-do 16677 (KR); KOO, Bonkon, Suwon-si, Gyeonggi-do 16677 (KR); LEE, Seungjae, Suwon-si, Gyeonggi-do 16677 (KR)
(74) Representative: Appleyard Lees IP LLP
(86) International application number: PCT/KR2022/009205
(87) International publication number: WO 2023/282524

(57) **Abstract**

Provided is a method, performed by an augmented reality (AR) device, of measuring a user's vision. The method includes obtaining a background image including an image of at least one physical object by capturing a front region of the AR device by using an outward facing camera included in the AR device, identifying an edge of the image of the at least one physical object in the background image, determining a first region for measuring the user's vision on the background image based on the identified edge, determining a second region corresponding to the determined first region on a display included in the AR device, outputting a virtual object for measuring the user's vision to the determined second region, obtaining a user input signal for vision measurement after outputting the virtual object, and calculating a vision prescription value of a user based on the obtained user input signal.

## Description

### Technical Field

The disclosure relates to an augmented reality (AR) device and method for measuring a user's vision and providing corrected vision to users with poor vision.

### Background Art

Augmented reality (AR) technology is a technology that synthesizes virtual objects or information with a real environment to make the virtual objects or information look like objects existing in a real physical environment. Modern computing and display technologies have enabled the development of systems for AR experiences, in which digitally reproduced images or parts thereof may be presented to users in such a way that they may be thought of as real or recognized as real.

As interest in AR technology increases, development of various technologies for implementing AR has been actively conducted. In particular, smart glasses may display a virtual object overlaid on a background image while directly recognizing an image of a real physical environment through a transparent display.

On the other hand, as most AR devices have a head mounted display (HMD) form, such an AR device is inconvenient to use while wearing glasses for vision correction. The vision before correction of a person wearing glasses for vision correction may appear complex due to myopia, hyperopia, astigmatism, or a combination thereof. When a user who needs vision correction uses an AR device without wearing glasses for vision correction, the user may not clearly recognize an image with respect to a real physical environment, and immersion in AR is reduced.

Accordingly, in order to provide a realistic AR service even to a user who does not wear separate glasses, a technology for accurately measuring a user's vision and providing vision correction is required.

### Disclosure

### Technical Problem

An embodiment of the disclosure may provide an augmented reality (AR) device and method determining a region where a virtual vision chart for measuring a user's vision is output in consideration of a real physical environment, thereby preventing an error in a vision measurement result due to external factors.

An embodiment of the disclosure may provide an AR device and method compensating for a vision measurement value according to a focal distance for recognizing a virtual vision chart by a user, thereby reducing an error between a user's actual vision and a measured vision.

An embodiment of the disclosure may provide an AR device and method controlling a focus of a variable focus lens such as a liquid crystal (LC) lens based on a measured vision, thereby providing a vision correction and a realistic AR service to a user.

The technical problem to be achieved by an embodiment of the disclosure is not limited to the technical problem as described above, and other technical problems may be inferred from the following embodiments.

### Technical Solution

As technical means for achieving the above-described technical problem, a method, performed by an augmented reality (AR) device, of measuring a user's vision may include obtaining a background image including an image of at least one physical object by capturing a front region of the AR device by using an outward facing camera included in the AR device, identifying an edge of the image of the at least one physical object in the background image, determining a first region for measuring the user's vision on the background image based on the identified edge, determining a second region corresponding to the determined first region on a display included in the AR device, outputting a virtual object for measuring the user's vision to the determined second region, obtaining a user input signal for vision measurement after outputting the virtual object, and calculating a vision prescription value of a user based on the obtained user input signal.

As technical means for achieving the above-described technical problem, an AR device measuring a user's vision may include an outward facing camera configured to obtain a background image including an image of at least one physical object by capturing a front region, an output unit configured to output a virtual object for measuring the user's vision, a user input unit configured to obtain a user input signal for vision measurement after outputting the virtual object, a storage storing a program including one or more instructions, and at least one processor configured to execute the one or more instructions stored in the storage. The at least one processor may be further configured to execute the one or more instructions to identify an edge of the image of the at least one physical object in the obtained background image, determine a first region for measuring the user's vision on the background image based on the identified edge, determine a second region corresponding to the determined first region on a display included in the output unit, output the virtual object to the determined second region, and calculate a vision prescription value of a user based on the obtained user input signal.

As technical means for achieving the above-described technical problem, a non-transitory computer-readable recording medium may have recorded thereon a program for executing at least one of embodiments of the disclosed method, on a computer.

### Description of Drawings

FIG. 1 is a schematic diagram of a method, performed by an augmented reality (AR) device, of measuring a user's vision, according to an embodiment of the disclosure.
FIG. 2 is a flowchart of a method, performed by an AR device, of measuring a user's vision, according to an embodiment of the disclosure.
FIG. 3 is a diagram for explaining an operation in which an AR device determines a position where a virtual object for measuring a user's vision is to be output, according to an embodiment of the disclosure.
FIG. 4 is a diagram for explaining an operation of identifying an edge of images of at least one physical object in a background image and determining a position where a virtual object for measuring a vision is to be output, according to an embodiment of the disclosure.
FIG. 5 is a diagram for explaining an operation of identifying an edge of images of at least one physical object in a background image and determining a position where a virtual object for measuring a vision is to be output, according to an embodiment of the disclosure.
FIG. 6 is a diagram for explaining an operation of determining a region for measuring a user's vision on a background image based on a depth map of the background image, according to an embodiment of the disclosure.
FIG. 7 is a diagram for explaining an operation of determining an output position of a virtual object for measuring a user's vision by using an object locking mechanism, according to an embodiment of the disclosure.
FIG. 8 is a diagram for explaining an operation of outputting guide indicators, according to an embodiment of the disclosure.
FIG. 9 is a diagram for explaining various operations for increasing the discrimination of a virtual object displayed on an AR device, according to an embodiment of the disclosure.
FIG. 10 is a diagram for explaining an operation of calculating a test vision compensation value based on focal distances to virtual objects, according to an embodiment of the disclosure.
FIG. 11 is a block diagram of an AR device according to an embodiment of the disclosure.
FIG. 12 is a diagram illustrating an AR device according to an embodiment of the disclosure.
FIG. 13 is a diagram for explaining an operation of controlling a variable focus lens and providing vision correction to a user, according to an embodiment of the disclosure.
FIG. 14 is a diagram illustrating an AR device according to an embodiment of the disclosure.

### Mode for Invention

Hereinafter, embodiments of the disclosure will be described in detail with reference to the accompanying drawings so that those of ordinary skill in the art may easily implement the embodiments of the disclosure. However, the disclosure may be embodied in many different forms and should not be construed as being limited to the embodiments set forth herein. Also, portions irrelevant to the description of the disclosure will be omitted in the drawings for a clear description of the disclosure, and like reference numerals will denote like elements throughout the specification.

The terms used herein are those general terms currently widely used in the art in consideration of functions in the disclosure, but the terms may vary according to the intentions of those of ordinary skill in the art, precedents, or new technology in the art. Also, in some cases, there may be terms that are optionally selected by the applicant, and the meanings thereof will be described in detail in the corresponding portions of the disclosure. Thus, the terms used herein should be understood not as simple names but based on the meanings of the terms and the overall description of the disclosure.

As used herein, the singular forms "a," "an," and "the" may include the plural forms as well, unless the context clearly indicates otherwise. Unless otherwise defined, all terms (including technical or scientific terms) used herein may have the same meanings as commonly understood by those of ordinary skill in the art of the disclosure.

Throughout the disclosure, when something is referred to as "including" an element, one or more other elements may be further included unless specified otherwise. Also, as used herein, terms such as "units" and "modules" may refer to units that perform at least one function or operation, and the units may be implemented as hardware or software or a combination of hardware and software.

Throughout the specification, when an element is referred to as being "connected" to another element, it may be "directly connected" to the other element or may be "electrically connected" to the other element with one or more intervening elements therebetween. In addition, the terms "comprises" and/or "comprising" or "includes" and/or "including" when used in this specification, specify the presence of stated elements, but do not preclude the presence or addition of one or more other elements.

The expression "configured to (or set to)" used herein may be used interchangeably with, for example, "suitable for", "having the capacity to", "designed to", "adapted to", "made to", or "capable of', according to situations. The expression "configured to (or set to)" may not only necessarily refer to "specifically designed to" in terms of hardware. Instead, in some situations, the expression "system configured to" may mean that the system is "capable of" along with other devices or components. For example, "a processor configured to (or set to) perform A, B, and C" may refer to a dedicated processor (e.g., an embedded processor) for performing a corresponding operation, or a general-purpose processor (e.g., a central processing unit (CPU) or an application processor) capable of performing a corresponding operation by executing one or more software programs stored in a memory.

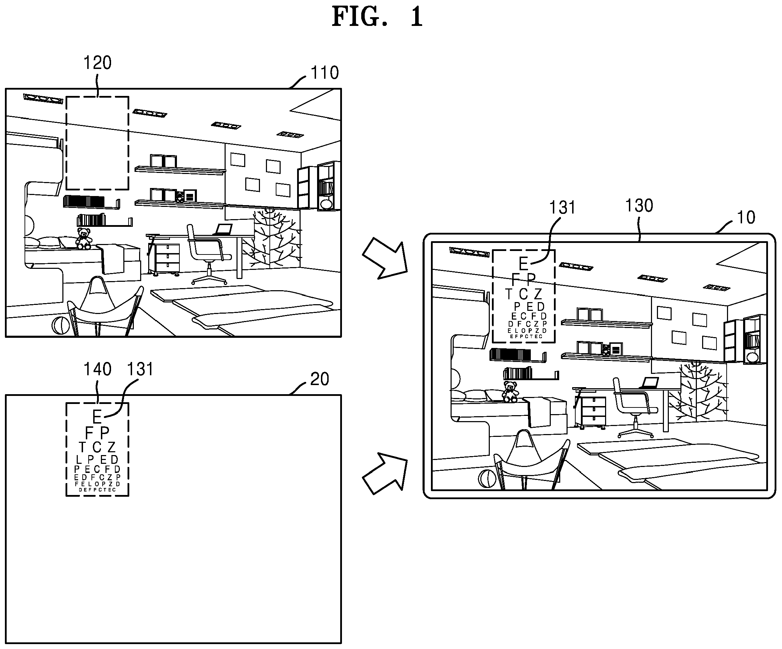

FIG. 1 is a schematic diagram of a method, performed by an augmented reality (AR) device, of measuring a user's vision according to an embodiment of the disclosure.

An AR device 10 is a device capable of expressing AR, and may display images including physical objects that exist in reality and virtual objects.

The AR device 10 may include, for example, AR glasses in the shape of glasses worn on the face of a user, a head mounted display (HMD), a virtual reality headset (VRH), or an AR helmet worn on the head. Meanwhile, the AR device 10 is not limited to the above examples, and may include all types of devices capable of providing AR services to the user.

Referring to FIG. 1, the AR device 10 may obtain a background image 110 of a real environment. For example, the background image 110 may be obtained by using an outward facing camera included in the AR device 10.

In an embodiment, the AR device 10 may determine, as a first region 120, a part of the background image 110 where no physical object exists or where an edge of the physical object is not complicated. The first region 120 may be a region for measuring the user's vision using the AR device 10 on the background image 110.

The AR device 10 may determine a second region 140 corresponding to the determined first region 120 on a display 20 in consideration of a user's gaze direction. A virtual object 131 for measuring the user's vision may be output to the determined second region 140. For example, the virtual object 131 for measuring the user's vision may be a text, a picture, or a vision test chart including one or more texts or pictures.

When the user wears the AR device 10 and looks at the real environment through the display 20 on which the virtual object 131 is displayed, the user may recognize a composite image 130 including the background image 110 of the real environment and the virtual object 131. In this regard, for example, the first region 120 on the background image 110 and the second region 140 on the display 20 corresponding to each other may indicate that the first region 120 and the second region 140 are accurately overlaid on the composite image 130.

According to an embodiment of the disclosure, the first region 120 for measuring the user's vision is determined on the background image 110, and when the AR device 10 is worn, the virtual object 131 for measuring the vision is output to the second region 140 on the display 20 to be accurately overlaid with the first region 120 in the user's gaze direction. In this case, the user may recognize that the virtual object 131 exists in the first region 120 on the background image 110.

As described above, a region where a virtual vision chart for measuring the user's vision is output is determined in consideration of a real physical environment, thereby preventing an error in a vision measurement result due to an external factor.

In addition, according to an embodiment of the disclosure, the accuracy of the vision measurement may be further increased by compensating for the eyesight measurement result according to a focal length for recognizing the virtual object 131. In addition, an AR service may be provided with a corrected vision based on the user's vision measured with high accuracy, thereby allowing the user to experience a more realistic AR.

Hereinafter, a method of determining a region for outputting a virtual object for measuring the user's vision, a method of compensating for a vision measurement result according to a focal distance to the virtual object, and a method of providing a vision correction based on the measured user's vision, etc. are described in more detail.

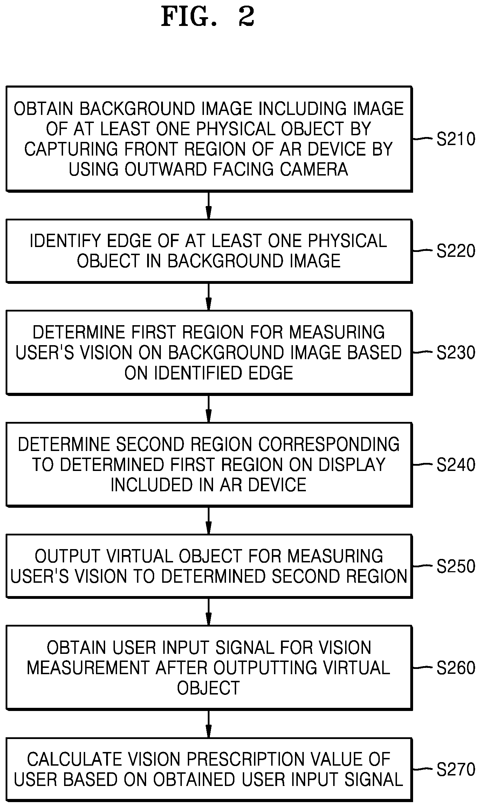

FIG. 2 is a flowchart of a method, performed by an AR device, of measuring a user's vision according to an embodiment of the disclosure.

In operation S210, the AR device may obtain a background image including an image of at least one physical object by capturing a front region of the AR device by using an outward facing camera. In an embodiment, the outward facing camera may obtain the background image by capturing the front region. In this case, an image captured at a specific time may be used to determine a region for measuring the user's vision in a real space.

In operation S220, the AR device may identify an edge of the image of at least one physical object in the background image. An operation of identifying the edge of the image may represent an operation of recognizing boundary lines of objects included in the image. An edge (boundary line) may indicate a place where the value of a pixel suddenly changes in an image. For example, an algorithm for edge extraction may be determined by the size of a gradient vector obtained by differentiating an image.

In an embodiment, the operation of extracting the edge included in the background image may use various edge extraction algorithms such as Sobel edge detection, Prewitt edge detection, Roberts edge detection, Compass edge extraction detection, Laplacian edge detection, Canny edge detection, etc.

In an embodiment, the operation of identifying the edge of the at least one physical object in the background image may determine, as the edge, a part whose intensity varies by a preset threshold value or more over adjacent pixels on the background image. For example, the edge may be determined even in a contour portion of the physical object, but may also be determined by a shape, pattern, curve, etc. within the physical object. For example, in the case of a picture frame, the picture frame is one physical object, but many edges may be identified according to a picture drawn in the picture frame. Also, for example, even though two different physical objects exist in the background image, when colors of the two objects are similar and a boundary between the two objects is not clearly visible, the edge may not be identified.

In operation S230, the AR device may determine a first region for measuring the user's vision on the background image based on the identified edge. In an embodiment, the AR device may determine a part of the background image in which no edge is detected as the first region for vision measurement. In an embodiment, a part having the largest area among parts where no edge is detected may be determined as the first region for vision measurement.

In an embodiment, when the size of the part in which no edge is detected is not large enough to output a virtual object for vision measurement, the first region may be determined over two regions having weak edge strength among adjacent edge-undetected regions. For example, even though an edge is identified between an image region corresponding to the sky and an image region corresponding to the sea, when the strength of the edge between the sky and the sea is weak compared to the strength of other edges included in the image, the first region may be determined over the image region corresponding to the sky and the image region corresponding to the sea.

Meanwhile, the operation of determining the first region for measuring the user's vision on the background image based on the identified edge is not limited to the above-described examples, and the first region may be determined by using various methods.

In step S240, the AR device may determine a second region corresponding to the determined first region on a display. The first region may be a region on the background image, and the second region may be a region on the display. In an embodiment, in the second region determined to correspond to the first region, when the user looks at the real space through the AR device, the first region included in the background image of the real space and the second region displayed on the display may be accurately overlaid and recognized as being in the same position. For example, a virtual object displayed on the second region of the display may be recognized as existing in the first region of the real space by the user wearing the AR device.

In an embodiment, the operation of determining the second region corresponding to the first region may use gaze direction information of the user obtained through an inward facing camera or an eye tracking (ET) camera included in the AR device. For example, a region recognized by the user wearing the AR device as being accurately overlaid on the first region may be determined as the second region by using the gaze direction information of the user.

In step S250, the AR device may output the virtual object for measuring the user's vision to the determined second region. In an embodiment, the display may include a transparent material. In an embodiment, the virtual object for measuring the user's vision may be one text, one picture, or a combination of one or more texts or pictures.

In operation S260, the AR device may obtain a user input signal for vision measurement after outputting the virtual object. In an embodiment, the user input signal may be a signal input as the user recognizes the output virtual object. In an embodiment, the user input signal may include a signal detected by a voice sensor such as a microphone, a signal detected by a touch sensor, a signal received through an input device, or various other signals.

In step S270, the AR device may calculate a vision prescription value of the user based on the obtained user input signal. The vision prescription value of the user may include information about the degrees of myopia, hyperopia, and astigmatism. In an embodiment, the AR device may provide the user with the calculated vision prescription value of the user. The calculated vision prescription value of the user may be used in an operation of providing vision correction to the user.

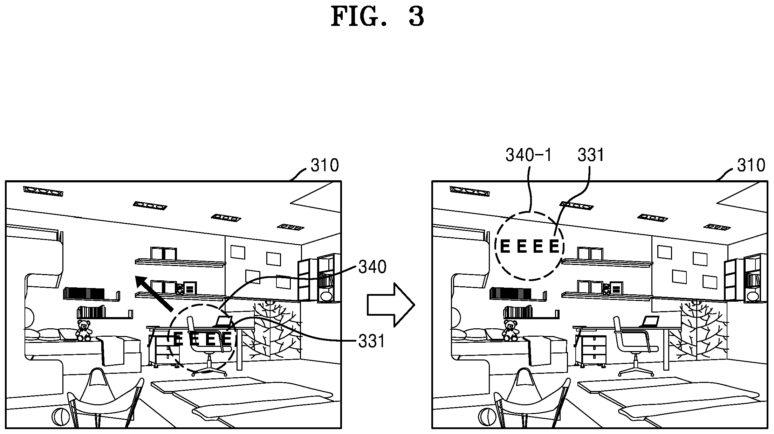

FIG. 3 is a diagram for explaining an operation in which an AR device determines a position where a virtual object 331 for measuring a user's vision is to be output according to an embodiment of the disclosure.

Referring to FIG. 3, a real environment 310 may include various physical objects. In an embodiment, a user may perform vision measurement within the real environment 310 including various physical objects. The AR device may include a transparent display. In an embodiment, the user wearing the AR device may view the virtual object 331 displayed on the transparent display while viewing the real environment 310 through the transparent display.

In an embodiment, region 1 340 in the real environment 310 may include one or more physical objects. For example, the region 1 340 may include a chair, a desk, a drawer, and a laptop computer. In an embodiment, region 2 340-1 in the real environment 310 may include only one physical object. For example, the region 2 340-1 may include only walls.

Referring to the left side of FIG. 3, when the virtual object 331 for measuring the user's vision is overlaid and displayed on the region 1 340, the recognition of the virtual object 331 may deteriorate. When edge analysis is performed on a background image corresponding to the real environment 310, a large number of edges may be included in the region 1 340. As such, when the virtual object 331 is displayed so that the edge corresponds to a complex region, the recognition of the virtual object 331 may be lowered due to physical objects included in the region.

Referring to the right side of FIG. 3, when the virtual object 331 for measuring the user's vision is overlaid and displayed on the region 2 340-1, the recognition of the virtual object 331 may be good. When edge analysis is performed on the background image corresponding to the real environment 310, no edge or a small amount of edges may be included in the region 2 340-1. As such, when the virtual object 331 is displayed to correspond to a region where the edge is not identified, physical objects do not exist or exist in the region only to the extent that they do not affect a user's field of view (FOV), and thus, the recognition of the virtual object 331 may increase.

The recognition of the virtual object 331 for vision measurement may affect a vision measurement result. For example, when the virtual object 331 is output on a region with complex edges, such as the region 1 340, a text that is sufficiently readable by the user may be misread. Accordingly, when the virtual object 331 is output on the region with complex edges, the vision may be measured lower than the actual user's vision.

According to an embodiment of the disclosure, for accuracy of vision measurement, the first region for measuring the user's vision may be determined on the background image 310 through edge analysis of the background image 310, and the determined first region may be used for vision measurement.

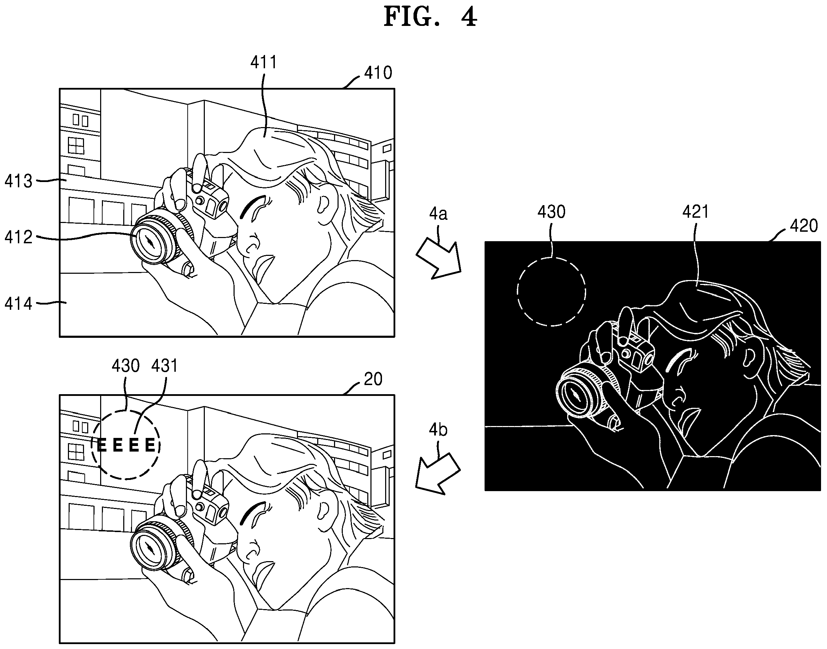

FIG. 4 is a diagram for explaining an operation of identifying an edge of images 411, 412, 413, and 414 of at least one physical object in a background image and determining a position where a virtual object 431 for measuring a vision is to be output according to an embodiment of the disclosure.

Referring to FIG. 4, a background image 410 may include the plurality of physical object images 411, 412, 413, and 414. For example, the background image 410 may include a person image 411, a camera image 412, a building image 413, and a land image 414.

In an embodiment, an AR device may identify an edge 421 of each of the physical object images 411, 412, 413, and 414 in the background image 410 (operation 4a). In an embodiment, the AR device may generate an edge map 420 by extracting the edge 421 included in the background image 410. The edge map 420 may include the at least one edge 421.

In an embodiment, in the operation of identifying the edge 421 of the physical objects in the background image 410, a part of the background image 410 whose intensity varies over adjacent pixels by a preset threshold value or more may be determined as the edge 421.

In an embodiment, no edge may be detected in an image of a specific physical object. For example, referring to FIG. 4, the edge 421 of the building image 413 may not be detected. The edge 421 may be detected when the intensity varies over adjacent pixels by the preset threshold value or more. In an embodiment, the edge 421 may not be detected because an intensity difference between pixels corresponding to the building image 413 is smaller than the preset threshold value. In this case, even on the actual background image 410, it may be difficult to distinguish a boundary or a pattern of the outside of the building with the naked eye. When it is difficult to distinguish the intensity difference between pixels with the naked eye, even though the virtual object 431 is displayed on the corresponding part, an error in vision measurement is less likely to occur. Accordingly, a region on the edge map 420 in which the edge 421 does not exist is determined as the first region 430 for measuring the user's vision, thereby preventing an error in vision measurement.

The AR device may determine a first region 430 for measuring the user's vision on the background image 410 based on the identified edge 420, determine a second region on the display 20 corresponding to the determined first region 430, and output the virtual object 431 for measuring the user's vision to the determined second region (operation 4b).

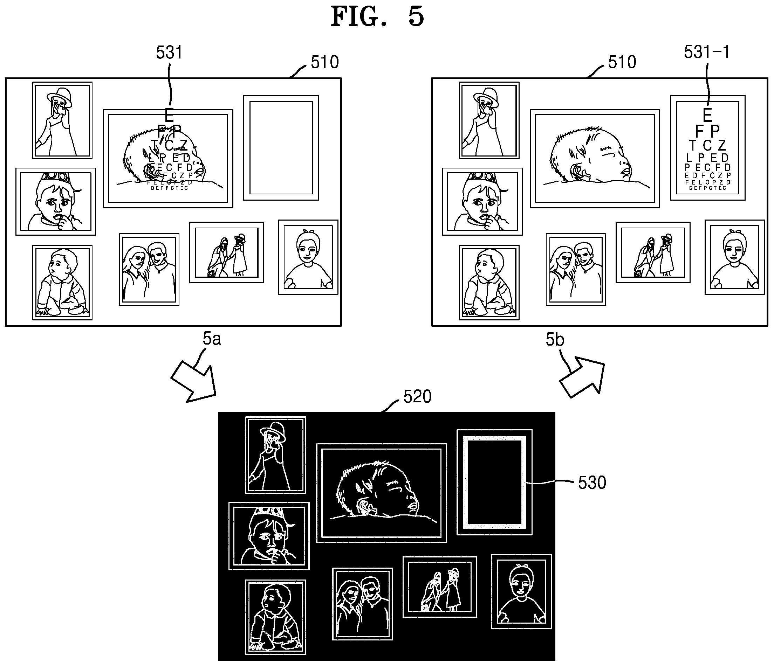

FIG. 5 is a diagram for explaining an operation of identifying an edge of images of at least one physical object in a background image 510 and determining a position where a virtual object 531 for measuring a vision is to be output according to an embodiment of the disclosure.

In an embodiment, the edge may be determined at a part corresponding to a contour of a physical object, but may also be determined by a shape, pattern, curve, etc. within the physical object. For example, in the case of a picture frame, the picture frame is one physical object, but many edges may be identified according to a pattern of a picture drawn inside the picture frame. In an embodiment, the edge may be determined based on values of pixels determined by the shape, position, pattern, color, etc. of the physical object in the background image 510.

Referring to operation 5a, in the background image 510 including a plurality of frame images, the edge may be determined by a border of the 'frame', which is the physical object, and the picture drawn inside the frame. In an embodiment, when the virtual object 531 is displayed on a region including a complex edge, the recognition of the virtual object 531 may deteriorate, and an error may occur in a vision measurement result.

An AR device may identify a region including no edge in an edge map 520 including determined edges, and determine the region as a first region 530 for vision measurement.

In operation 5b, the AR device may adjust a position of a virtual object 531-1 to correspond to the determined first region 530. When the virtual object 531-1 is displayed on the region including no edge, the virtual object 531-1 may be easily recognized within a user's vision range, and accurate vision measurement is possible.

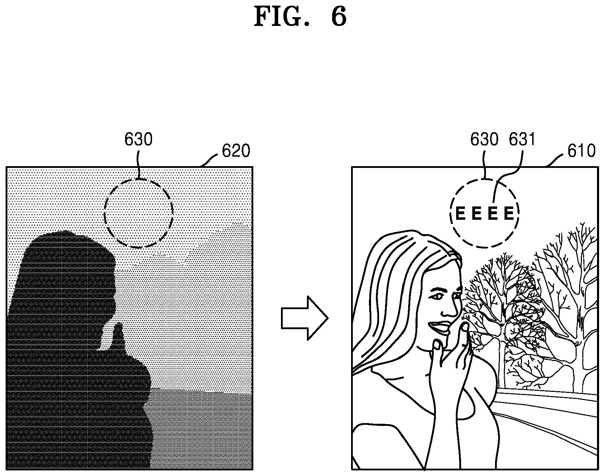

FIG. 6 is a diagram for explaining an operation of determining a region for measuring a user's vision on a background image 610 based on a depth map 620 of the background image 610 according to an embodiment of the disclosure.

In an embodiment, an AR device may obtain the depth map 620 of the background image 610 by using a depth sensor, and identify a depth value and shape of at least one physical object included in the background image 610 based on the obtained depth map 620. In an embodiment, the AR device may provide a first region 630 for measuring the user's vision on the background image 610 based on the depth value and shape of the at least one physical object and edges identified through various edge extraction methods.

In an embodiment, the AR device may select a flat region having a relatively constant focal length with respect to a virtual object 631 to be displayed by using depth information, and select a region in which the virtual object 631 may be easily recognized or identified by using edge information because no physical object exists. In an embodiment, when a region is selected based on both the depth information and the edge information, a region having a relatively constant pixel value and a relatively constant focal length may be selected as the first region 630 for vision measurement.

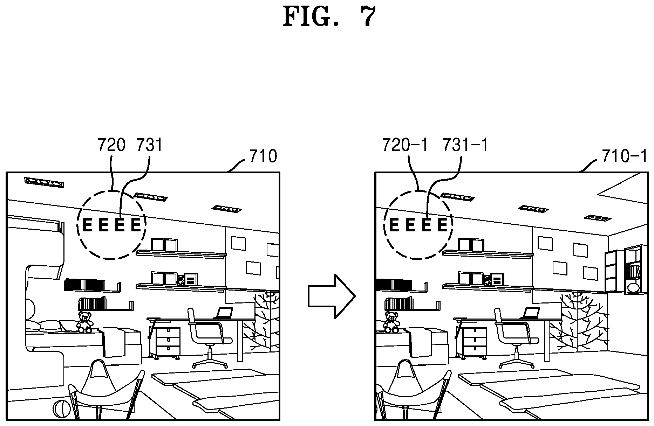

FIG. 7 is a diagram for explaining an operation of determining an output position 720 of a virtual object 731 for measuring a user's vision by using an object locking mechanism according to an embodiment of the disclosure.

The object locking mechanism is a mechanism for fixing relative positions of a physical object and a virtual object in a virtual reality (VR) or AR display.

An AR device according to an embodiment of the disclosure may use the object locking mechanism to determine a second region on a display corresponding to a first region of a background image 710. In an embodiment, a user wearing the AR device may change a pose or gaze at a different place while vision measurement is in progress.

In an embodiment, the relative position of the virtual object 731 with respect to the background image 710 may change according to a movement of the user. When the user moves, a position of the AR device being worn is changed, and thus, a position of a display included in the AR device is changed. As the position of the display is changed, the alignment of the first region on the background image 710 corresponding to the second region on the display may be misaligned.

When the alignment of the first region on the background image 710 and the second region on the display are misaligned, the first region and the second region may not be accurately overlaid in a user's gaze direction, and a virtual object displayed on the second region may move away from the first region on the background image 710 to a region including many edges. In this case, the discrimination and recognition of the virtual object may be lowered, and the accuracy of a vision measurement result may be reduced.

In an embodiment, the AR device may adjust the second region so that the virtual object for measuring the user's vision is displayed on the display to overlay on the certain first region, through the object locking mechanism. Accordingly, even though the user moves while the vision measurement is in progress, the relative positions of the virtual object 731 and the background image 710 may not change.

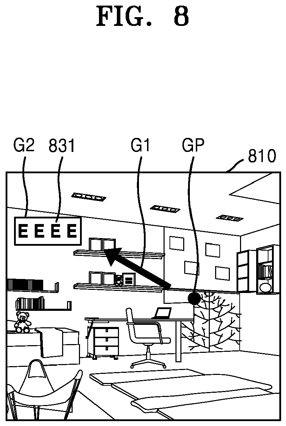

FIG. 8 is a diagram for explaining an operation of outputting guide indicators G1 and G2 according to an embodiment of the disclosure.

Referring to FIG. 8, an AR device may recognize a user's gaze point (GP). In an embodiment, the user's GP may be recognized through an inward facing camera or an ET camera included in the AR device.

In an embodiment, when it is determined that the recognized user's GP is not toward a virtual object 831, the AR device may output the guide indicators G1 and G2 to a display.

Referring to FIG. 8, the guide indicators G1 and G2 may include various types of indicators capable of guiding a user's gaze movement and emphasizing a position of the virtual object 831, such as an arrow G1 pointing toward the virtual object 831 in the recognized user's GP and a box G2 surrounding the virtual object 831.

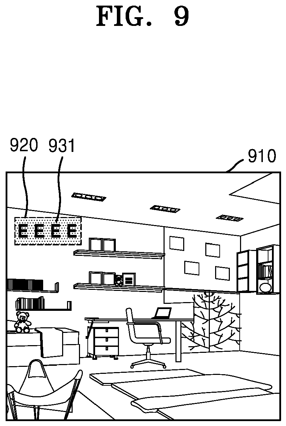

FIG. 9 is a diagram for explaining various operations for increasing the discrimination of a virtual object 931 displayed on an AR device according to an embodiment of the disclosure.

In an embodiment, the AR device may adjust a color of the virtual object 931 to increase the discrimination of the displayed virtual object 931. In an embodiment, the AR device may identify a color of a first region 920 on a background image 910 on which the virtual object 931 is to be overlaid. The AR device may then determine the color of the virtual object 931 based on the identified color of the first region 920.

In an embodiment, the greater the color contrast between two adjacent objects, the easier it is to distinguish the objects. That is, the greater the color contrast between the objects, the higher the discrimination between the objects. In an embodiment, the AR device may determine the color of the virtual object 931 to have a maximum contrast with the color of the first region 920.

In an embodiment, the AR device may differently determine the color of the virtual object 931 according to whether the user is color-blind or color-weak. In an embodiment, the AR device may determine the color of the virtual object 931 through newly obtained or pre-stored user information so that the user may better identify the virtual object 931.

In an embodiment, the AR device may reduce the brightness of pixels included in the second region 920 except for pixels in which the virtual object 931 is output on a display. In an embodiment, when pixels around a region where the virtual object 931 is displayed are corrected to be dark, the region of the virtual object 931 may be highlighted so that the discrimination of the virtual object 931 may be further enhanced.

In an embodiment, at least one of the size and number of virtual objects 931 for measuring the user's vision may be determined based on the area of the first region 920 for measuring the user's vision on the background image 910. For example, the number, sizes, and gaps of the virtual objects 931 may be adjusted according to the area of a region where the virtual objects 931 are displayed.

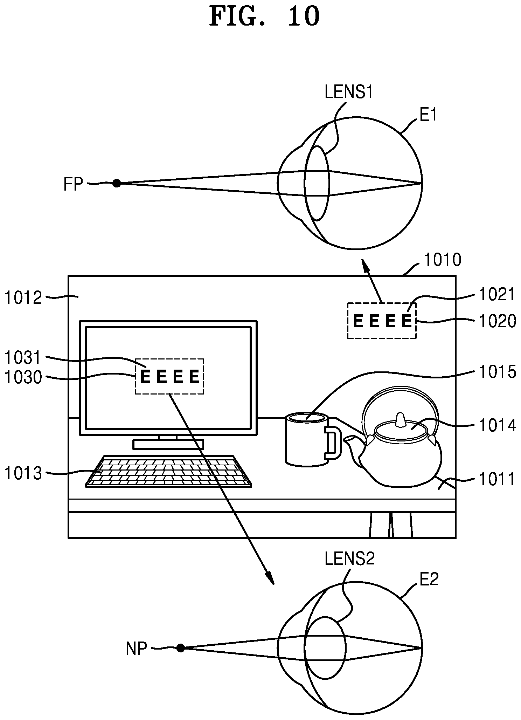

FIG. 10 is a diagram for explaining an operation of calculating a test vision compensation value based on focal distances to first and second virtual objects 1021 and 1031 according to an embodiment of the disclosure.

In an embodiment, an AR device may identify the focal distances from user's eyes E1 and E2 to the virtual objects 1021 and 1031, and calculate the test vision compensation value based on the identified focal distances.

The human eye reflexively performs two motions when viewing a real object (physical object). One is a vergence (convergence) motion, in which both eyes converge toward an object. In the vergence (convergence) motion, an optical axis of both eyes rotates according to a distance between the human eye and the object. The other is a focus adjustment (accommodation) motion, which the lens of the eye focuses so that an object is clearly visible. In the focus adjustment (accommodation) motion, a thickness of the lens is adjusted according to the distance between the human eye and the object.

In general, when a user gazes at a VR image or a virtual object output from an AR device, the user may experience a vergence-accommodation conflict (VAC) phenomenon. For example, when a distance from a VR device to a physical object is d1, the VR device may display a virtual object as if it is located at the distance d1. At this time, because the user views the virtual object as if it is located at the distance d1, a vergence distance at which both eyes of the user converge is d1. On the other hand, because the virtual object is actually displayed on a display of the VR device, a focal distance between both eyes of the user is a distance d2 from the user's eyes to the display. In this case, the vergence distance and the focal distance conflict, and the user of the VR device may feel dizzy or motion sick. Meanwhile, such a VAC problem may be solved through digital holography technology or a focus adjustment lens.

In the AR device according to an embodiment of the disclosure, a virtual object output on the display corresponds to a 2D text or image for vision measurement, not a 3D object. Accordingly, when the user views a composite image of a background image and a virtual object, the user may recognize the virtual object as being 'printed' on real objects in the background image or projected onto a real space. That is, the user's eyes gazing a 2D virtual object perform the vergence (convergence) and focus adjustment motions in the same way as when looking at a space where the virtual object is actually projected or an actual physical object located in the space.

Referring to FIG. 10, for example, a background image 1010 may include a desk image 1011, a sky image 1012, a computer image 1013, a teapot image 1014, and a cup image 1015. In an embodiment, when the user gazes at a virtual object 1021 projected on the sky region 1012 on the background image 1010, the user's eyes E1 may perform the vergence (convergence) and focus adjustment motions in the same way as when looking at a space 1020 where the virtual object 1021 is actually projected. In an embodiment, when the user gazes at a virtual object 1031 projected on the computer image 1013 on the background image 1010, the virtual object 1031 may be recognized as printed or displayed on a computer that is a real object in the background image 1010, and the user's eyes E2 may perform the vergence (convergence) and focus adjustment motions in the same way as when looking at a space 1030 where the virtual object 1031 is actually projected.

When the user views the background image 1010 through the AR device, and focuses on the computer image 1013 at a close distance and focuses on the sky image 1012 at a long distance, the thickness of the lens is different Therefore, when the vision is measured by using the virtual object 1031 projected on the computer image 1013 and the vision is measured by using the virtual object 1021 projected on the sky image 1012, even though measurement values are the same, there may be a difference in the vision due to a difference in the thickness of the lens. In an embodiment, an error between the user's actual vision and the measured vision may be reduced by compensating for a vision prescription value in consideration of a change in the lens.

For example, when the user focuses FP on the sky image 1012, a lens LENS1 included in the user's eye E1 has a relaxed state so as to be able to view a long distance. In an embodiment, the first virtual object 1021 for measuring the user's vision may be overlaid and displayed on the sky image 1012. In this case, the user reads the first virtual object 1021 in a state in which the lens LENS1 is relaxed so as to be able to view a long distance.

In another example, when the user focuses NP on the computer image 1013, a lens LENS2 included in the user's eye E2 has a swollen state so as to be able to view a short distance. In an embodiment, the second virtual object 1031 for measuring the user's vision may be overlaid and displayed on the computer image 1013. In this case, the user reads the second virtual object 1031 in a state where the lens LENS2 is swollen so as to be able to view a short distance.

Therefore, a measurement result is different between a case where the vision is measured by using the first virtual object 1021 and a case where the vision is measured by using the second virtual object 1031. For example, when the vision is measured by using the first virtual object 1021 and the vision is 1.2, the user's vision may be 1.2, but the vision is measured by using the second virtual object 1031 and the vision is 1.2, the user's actual vision may be lower than 1.2. (the user may not view letters further away)

Accommodation power of the lens of the user's eyes looking at the virtual object may be calculated from the focal length of the virtual object. In an embodiment of the disclosure, a virtual object may be 'printed' on real objects in a background image or recognized as being projected onto a real space, and thus, a focal length of the virtual object may be regarded as a distance to an actual physical object corresponding to a position in which the virtual object is projected.

For example, the distance to the actual physical object corresponding to the position in which the virtual object is projected may be measured through a depth sensor or a distance measuring sensor, or may be measured through an ET sensor. The depth sensor may include, for example, a time-of-flight (ToF) sensor, a stereo matching sensor using two cameras, or a structured light sensor. A method of measuring the distance to the actual physical object through the ET sensor may include a method of finding out gaze directions of user's left and right eyes through the ET sensor, determining a point where the gaze directions of user's left and right eyes intersect as a position where the object exists, and calculating a distance to the point.

In an embodiment, test vision compensation value (accommodation power (AP)) based on the focal distance of the virtual object may be determined based on a reciprocal number 1/D of an identified focal distance D. In an embodiment, the AR device may compensate for a vision prescription value calculated through the determined AP. For example, an operation of compensating for the vision prescription value calculated through the AP may compensate for the vision prescription value by subtracting the AP from the vision prescription value when the virtual object is displayed at the focal distance D within 6 m. For example, when the virtual object is displayed at the focal distance D greater than 6 m, the vision prescription value may be compensated for by adding the AP to the vision prescription value.

In an embodiment, an operation of identifying the focal distance from the user's eye may include an operation of identifying a physical object corresponding to a first region on a background image corresponding to a second region on a display and an operation of identifying a focal distance from the user's eyes to the identified physical object by using at least one of a LIDAR, a depth sensor, or an ET sensor included in the AR device.

According to an embodiment of the disclosure, an error between a user's actual vision and measured vision may be reduced by compensating for a vision prescription value according to a focal distance for recognizing a virtual vision chart by the user.

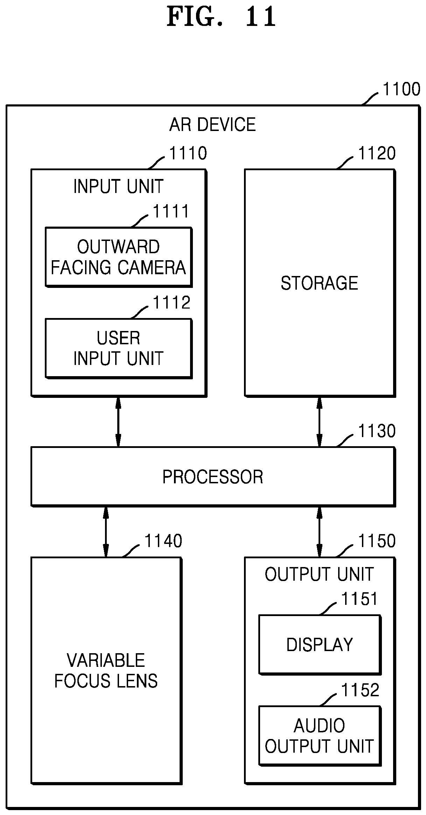

FIG. 11 is a block diagram of an AR device 1100 according to an embodiment of the disclosure.

Referring to FIG. 11, the AR device 1100 according to an embodiment of the disclosure may include an input unit 1110, a storage 1120, a processor 1130, a variable focus lens 1140, and an output unit 1150. The input unit 1110 may include an outward facing camera 1111 and a user input unit 1112. The output unit 1150 may include a display 1151 and an audio output unit 1152. All of components shown in FIG. 11 are not indispensable components of the AR device 1100. The AR device 1100 may be implemented by more components than those illustrated in FIG. 11, or the AR device 1100 may be implemented by fewer components than those illustrated in FIG. 11.

The outward facing camera 1111 may obtain a background image including an image of at least one physical object by capturing a front region of the AR device 1100.

The output unit 1150 may output a virtual object for measuring the user's vision.

The user input unit 1112 may obtain a user input signal for vision measurement after outputting the virtual object.

The storage 1120 may store a program to be executed by the processor 1130 to be described below in order to control the operation of the AR device 1100. The storage 1120 may store a program including at least one instruction for controlling the operation of the AR device 1100. Instructions and program codes readable by the processor 1130 may be stored in the storage 1120. In an embodiment, the processor 1130 may be implemented to execute instructions or codes of the program stored in the storage 1120. The storage 1120 may store data input to or output from the AR device 1100.

The storage 1120 may include at least one type of storage medium from among a flash memory, a hard disk, a multimedia card micro type storage medium, a card type memory (for example, SD or XD memory), a random access memory (RAM), a static RAM (SRAM), a read-only memory (ROM), an electrically erasable programmable ROM (EEPROM), a programmable ROM (PROM), a magnetic memory, a magnetic disk, and an optical disk.

Programs stored in the storage 1120 may be classified into a plurality of modules according to their functions.

The processor 1130 may control overall operations of the AR device 1100. The processor 1130 may perform operations according to an embodiment of the disclosure. For example, the processor 1130 may control all of the input unit 1110, the storage 1120, the variable focus lens 1140, and the output unit 1150 by executing the programs stored in the storage 1120.

The processor 1130 may include hardware components that perform arithmetic, logic, and input/output operations and signal processing. The processor 1130 may include, for example, but not limited to, at least one of a central processing unit, a microprocessor, a graphics processing unit, application specific integrated circuits (ASICs), DSPs, digital signal processing devices (DSPDs), programmable logic devices (PLDs), or field programmable gate arrays (FPGAs).

In an embodiment, the processor 1130 may execute the one or more instructions stored in the storage 1120 to identify an edge of an image of at least one physical object in an obtained background image, determine a first region for measuring the user's vision on the background image based on the identified edge, determine a second region corresponding to the determined first region on a display included in the output unit 1150, output a virtual object to the determined second region, and calculate a vision prescription value of the user based on a obtained user input signal.

In an embodiment, the processor 1130 may execute the one or more instructions stored in the storage 1120 to identify a focal distance from the user's eye to a virtual object, and calculate a test vision compensation value based on the identified focal distance, and compensate for the calculated vision prescription value based on the test vision compensation value.

In an embodiment, the processor 1130 may execute the one or more instructions stored in the storage 1120 to identify a color of the first region, and determine a color of the virtual object for measuring the user's vision to have a maximum contrast with the identified color of the first region, thereby increasing the discrimination of the virtual object.

In an embodiment, the processor 1130 may execute the one or more instructions stored in the storage 1120 to determine a second region so that the virtual object for measuring the user's vision is displayed on the display to be overlaid on the certain first region, through the object locking mechanism.

The variable focus lens 1140 is a lens having a variable focus. For example, the variable focus lens 1140 may include a liquid crystal (LC) lens, a liquid membrane lens, a liquid wetting lens, or an Alvarez lens. For example, the LC lens is a lens having a variable refractive index controlled by an electrical signal. The AR device 1100 may control the focus of the variable focus lens 1140 based on the calculated vision prescription value of the user.

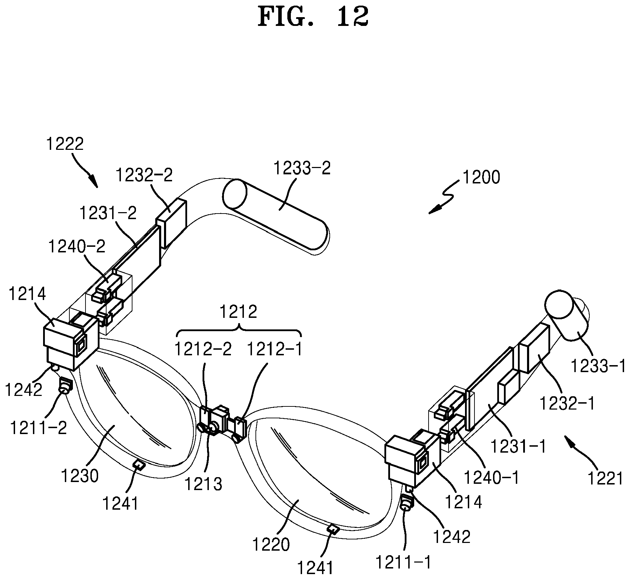

FIG. 12 is a diagram illustrating an AR device according to an embodiment of the disclosure.

Referring to FIG. 12, an AR device 1200 capable of measuring a user's vision and providing correction of the vision based on the measured vision is illustrated. The AR device 1200 is a device capable of displaying AR, and may include generally AR glasses in the shape of glasses worn on the face of a user, a HMD, a VRH, or an AR helmet worn on the head. In the case of the HMD, a super-large screen may be provided to the user by placing a display in front of the user's eyes, and a realistic virtual world may be provided as the screen moves according to a user's movement.

In an embodiment, a user may wear the AR device 1200, capable of displaying visual extended reality content. The AR device 1200 may include an audio module capable of providing audio extended reality content to the user. In an embodiment, AR device 1200 may include one or more cameras capable of capturing an image and video of an environment. The AR device 1200 may include an eye tracking system to determine a vergence distance of the user. In an embodiment, the AR device 1200 may include a lightweight HMD (e.g., goggles, glasses, visor, etc.). In an embodiment, the AR device 1200 may include a non-HMD device, such as a lightweight and portable display device or one or more laser projection glasses (e.g., glasses capable of projecting a low-powered laser on the user's retina to project and display an image or depth content to the user).

In an embodiment, the AR device 1200 may provide an AR service that outputs at least one virtual object to appear overlaid on a region determined as a user's FOV. For example, the region determined to be the user's FOV is a region determined to be perceptible by a user wearing the AR device 1200 through the AR device 1200, and may be a region including the entire display of the AR device 1200 or at least a part of the display. In an embodiment, the AR device 1200 may include a plurality of transparent members (e.g. a first display 1220 and a second display 1230) respectively corresponding to both eyes of the user.

In an embodiment, the AR device 1200 may include a display module 1214, a camera, an audio output unit, and support units 1221 and 1222.

The camera may capture an image corresponding to the user's FOW or measure a distance to an object. In an embodiment, the camera may be used for head tracking and spatial recognition. Also, the camera may recognize a user's movement.

In an embodiment, the camera may further include an 'ET camera 1212', in addition to a camera 1213 used for detecting an image corresponding to the user's FOV, that is, motion of an object, or spatial recognition. In an embodiment, the ET camera 1212 may be used to detect and track the pupil of the user. The ET camera 1212 may be used for adjusting the center of a virtual image projected on the AR device 1200 to be positioned in a direction in which the eyes of the user wearing the AR device 1200 gaze. For example, a global shutter (GS) camera may be used in the ET camera 1212 to detect the pupil and track a fast pupil movement without a delay. The ET camera 1212 may separately include a left-eye camera 1212-1 and a right-eye camera 1212-2.

In an embodiment, the display module 1214 may include the first display 1220 and the second display 1230. A virtual object output through the display module 1214 may include information related to an application program executed on the AR device 1200 or information related to an external object located in a real space corresponding to a region determined as the user's FOV. For example, the AR device 1200 may check an external object included in at least a part corresponding to the region determined as the user's FOV among image information related to the real space obtained through the camera 1213. The AR device 1200 may output a virtual object related to the external object checked in the at least part through the region determined as the user's FOV among display regions of the AR device 1200. The external object may include objects existing in the real space.

In an embodiment, the first and second displays 1220 and 1230 each may include a condensing lens or a waveguide in a transparent member. For example, the transparent member may be formed from a glass plate, plastic plate, or polymer, and may be manufactured completely transparent or translucent. In an embodiment, the transparent member may include a first transparent member 1230 facing the right eye of the user wearing the AR device 1200 and a second transparent member 1220 facing the left eye of the user. When the first and second displays 1220 and 1230 are transparent, the displays may be disposed at a position facing the user's eyes to display a screen.

The waveguide may deliver light generated from a light source of the displays to the user's eyes. For example, the waveguide may be at least partially positioned on a portion of the transparent members (e.g. the first display 1220 and the second display 1230). According to an embodiment, light emitted from the displays may be incident to one end of the waveguide, and the incident light may be transmitted to the user's eyes through total internal reflection within the waveguide. The waveguide may be manufactured from a transparent material such as glass, plastic, or polymer, and may include a nanopattern formed on an inner or outer surface, for example, a polygonal or curved grating structure. In an embodiment, the incident light may be propagated or reflected inside the waveguide by the nanopattern and provided to the user's eyes. In an embodiment, the waveguide includes at least one of at least one diffractive element (e.g., a diffractive optical element (DOE), a holographic optical element (HOE)) or a reflective element (e.g., a mirror). In an embodiment, the waveguide may guide a display light emitted from a light source unit to the user's eyes by using the at least one diffractive element or the reflective element.

In an embodiment, the first and second displays 1220 and 1230 each may include a display panel or lens (e.g., glass). For example, the display panel may include a transparent material such as glass or plastic. In an embodiment, the displays may include a transparent device, and the user may perceive a real space behind the displays by passing through the displays. The displays may display the virtual object on at least a partial region of the transparent device so that it looks like the virtual object is added to at least a part of the real space.

In an embodiment, the support units 1221 and 1222 may include respectively printed circuit boards (PCBs) 1231-1 and 1231-2 transmitting electrical signals to each component of the AR device 1200, audio speakers 1232-1 and 1232-2 outputting signals or batteries 1233-1 and 1233-2 supplying power. For example, in the glasses-type AR device 1200, the support units 1221 and 1222 may be disposed on temple parts of the glasses. The support units 1221 and 1222 may respectively include hinge units 1240-1 and 1240-2 coupled to the main body of the AR device 1200. The speakers 1232-1 and 1232-2 include a first speaker 1232-1 transmitting an audio signal to the user's left ear and a second speaker 1232-2 transmitting an audio signal to the user's right ear.

Referring to FIG. 12, the AR device 1200 may include a microphone 1241 receiving a user's voice and ambient sounds. In addition, the AR device 1200 may include at least one illumination LED 1242 to increase accuracy of at least one camera (e.g., the ET camera 1212, the outward facing camera 1213, or recognition cameras 1211-1 and 1211-2). For example, the illumination LED 1242 may be used as an auxiliary means for increasing accuracy when photographing a user's pupil with the ET camera 1212, and may use an IR LED of an infrared wavelength rather than a visible light wavelength. For example, the illumination LED 1242 may be used as an auxiliary means when it is not easy to detect a subject due to a dark environment when photographing a user's gesture by using the recognition cameras 1211-1 and 1211-2.

According to an embodiment, the display module 1214 may include a first light guide plate corresponding to a left eye and a second light guide plate corresponding to a right eye, and provide visual information to the user through the first light guide plate and the second light guide plate. According to an embodiment, the display module 1214 may include a display panel and a lens (e.g., a glass lens or an LC lens). The display panel may include a transparent material such as glass or plastic.

According to an embodiment, the display module 1214 may include a transparent device, and the user may pass through the display module 1214 and perceive a real space which is a rear surface of the display module 1214 in front of the user. The display module 1214 may display the virtual object on at least a partial region of the transparent device so that it looks like the virtual object is added to at least a part of the real space.

In an embodiment, the AR device 1200 may determine an external object included in at least a part corresponding to a region determined as the user's FOV among image information related to the real space obtained through the outward facing camera 1213. The AR device 1200 may output (or display) a virtual object related to the external object checked in the at least part through a region determined as the user's FOV among display regions of the AR device 1200. The external object may include objects existing in the real space. According to various embodiments, a display region where the AR device 1200 displays a virtual object may include a part of the display module 1214 (e.g., at least a portion of a display panel). According to an embodiment, the display region may correspond to at least a part of each of the first light guide plate and the second light guide plate.

According to an embodiment, the AR device 1200 may measure a distance to a physical object located in a front direction of the AR device 1200 by using the outward facing camera 1213. The outward facing camera 1213 may include a high resolution camera such as a high resolution (HR) camera and a photo video (PV) camera.

The AR device 1200 according to an embodiment of the disclosure is not limited to the above-described configuration, and may include various components in various positions and in various numbers.

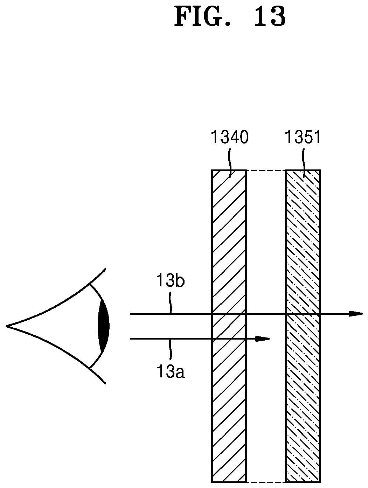

FIG. 13 is a diagram for explaining an operation of controlling a variable focus lens 1340 and providing a vision correction to a user according to an embodiment of the disclosure.

The variable focus lens 1340 is a lens having a variable focus. For example, the variable focus lens 1340 an LC lens, a liquid membrane lens, a liquid wetting lens, or an Alvarez lens.

In an AR device according to an embodiment of the disclosure, the variable focus lens 1340 may be disposed between the user's eyes and a display 1351. In an embodiment, the AR device may control the variable focus lens 1340 included in the AR device based on a calculated vision prescription value of a user.

Referring to FIG. 13, the user wearing the AR device may view a virtual object displayed on the display 1351 through the variable focus lens 1340 (13a), and view a space of reality through the variable focus lens 1340 and the transparent display 1351.

An embodiment of the disclosure may control the focus of the variable focus lens 1340 based on the measured vision, thereby providing vision correction to the user, and providing a realistic AR service through the corrected vision.

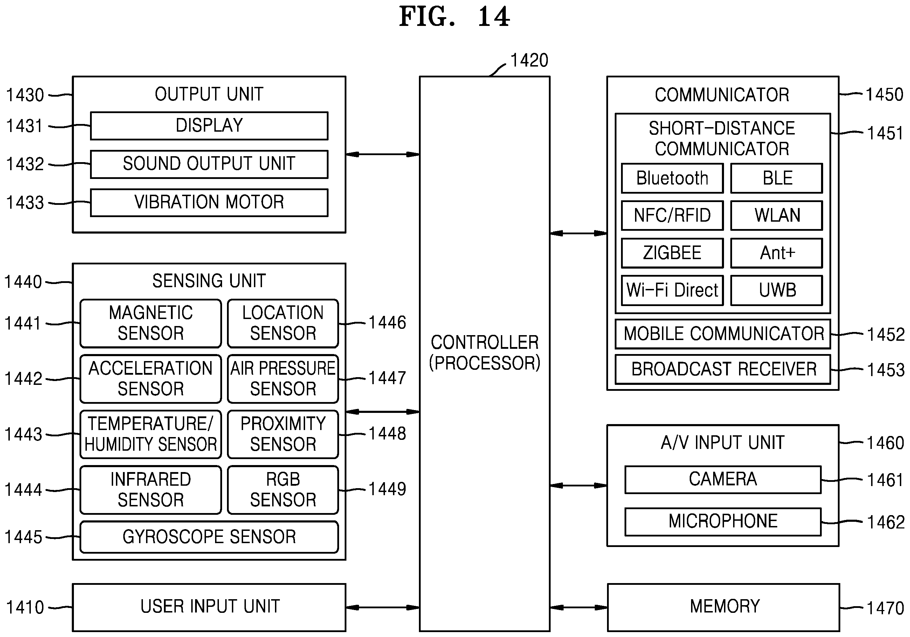

FIG. 14 is a diagram illustrating an AR device (e.g., 1100 of FIG. 11) according to an embodiment of the disclosure.

Referring to FIG. 14, the AR device may include a user input unit 1410, an output unit 1430, a controller 1420, a sensing unit 1440, a communicator 1450, an audio/video (A/V) input unit 1460, and a memory 1470.

The user input unit 1410 is a means through which a user inputs data for controlling the AR device. For example, the user input unit 1410 may include a touch pad (a touch capacitance method, a pressure-resistive layer method, an infrared sensing method, a surface ultrasonic conductive method, an integral tension measuring method, a piezo effect method, etc.) or a microphone, but is not limited thereto.

The output unit 1430 may output an audio signal, video signal, or vibration signal, and may include a display 1431, a sound output unit 1432, and a vibration motor 1433.

The display 1431 displays and outputs information processed by the AR device. For example, the display 1431 may display an image of a virtual object.

The display 1431 may include, for example, at least one of a liquid crystal display, a thin film transistor-liquid crystal display, an organic light-emitting diode display, a flexible display, a three-dimensional (3D) display, or an electrophoretic display.

The audio output unit 1432 outputs audio data received from the communicator 1450 or stored in the memory 1470. The vibration motor 1433 may output a vibration signal.

The controller 1420 typically controls overall operations of the AR device. In an embodiment, the controller 1420 may be implemented similarly to the processor 1130 of FIG. 11 described above. For example, the controller 1420 may execute programs stored in the memory 1470 to generally control the user input unit 1410, the output unit 1430, the sensing unit 1440, the communicator 1450, and the A/V input unit 1460. The controller 1420 may perform various operations of the AR device of FIGS. 1 to 12, by controlling the user input unit 1410, the output unit 1430, the sensing unit 1440, the communicator 1450, and the A/V input unit 1460.

The sensing unit 1440 may sense a state of the AR device or a state around the AR device, and transmit sensed information to the controller 1420.

The sensing unit 1440 may include at least one of a magnetic sensor 1441, an acceleration sensor 1442, a temperature/humidity sensor 1443, an infrared sensor 1444, a gyroscope sensor 1445, a location sensor (e.g., a GPS) 1446, an air pressure sensor 1447, a proximity sensor 1448, or an RGB sensor (an illuminance sensor) 1449, but is not limited thereto. The function of each sensor may be intuitively inferred from its name, and thus, a detailed description thereof is omitted.

The communicator 1450 may include one or more components for communication with other electronic devices. For example, the communicator 1450 may include a short-distance communicator 1451, a mobile communicator 1452, and a broadcast receiver 1453.

The short-range wireless communicator 1451 may include a Bluetooth communicator, a Bluetooth Low Energy (BLE) communicator, a near field communicator, a WLAN communicator, a WLAN (WiFi) communicator, a Zigbee communicator, an infrared data association (IrDA) communicator, a Wi-Fi direct (WFD) communicator, an ultra wideband (UWB) communicator, an Ant+ communicator, etc., but is not limited thereto.

The mobile communicator 1452 transmits and receives a radio signal to and from at least one of a base station, an external terminal, or a server on a mobile communication network. Here, the radio signal may include various types of data according to a speech call signal, a video call signal, or a text/multimedia message transmission/reception. In an embodiment, the AR device functions as a display device for other connected electronic devices, and the AR device itself may function as an independent mobile communication terminal. In this case, the communicator 1450 of the AR device may include both the short-range communicator 1451 and the mobile communicator 1452, and may operate as the independent mobile communication terminal through the mobile communicator 1452 even when not connected to other electronic devices.

The broadcast receiver 1453 may receive a broadcast signal and/or broadcast-related information from outside through a broadcast channel. The broadcast channel may include a satellite channel and a terrestrial channel. In an embodiment, the AR device may not include the broadcast receiver 1453.

The A/V input unit 1460 is for inputting an audio signal or a video signal, and may include a camera 1461 and a microphone 1462. The camera 1461 may obtain an image frame such as a still image or a moving image through an image sensor in a video communication mode or a photographing mode. An image captured through an image sensor may be processed through the controller 1420 or a separate image processing unit (not shown).

The image frame processed by the camera 1461 may be stored in the memory 1470 or transmitted to the outside through the communicator 1450. Two or more cameras 1461 may be provided according to the configuration of the AR device.

The microphone 1462 receives an external sound signal and processes the received signal as electrical speech data. For example, the microphone 1462 may receive a sound signal from an external device or a speaker. The microphone 1462 may use various noise removal algorithms to remove noise generated in a process of receiving an external sound signal.

The memory 1470 may store programs for processing and control by the controller 1420 and may store data input to or output from the AR device. The memory 1470 may include at least one type storage medium of a flash memory type, a hard disk type, a multimedia card micro type, a card type memory (e.g., SD orXD memory), RAM (Random Access Memory), SRAM (Static Random Access Memory), ROM (Read Only Memory), EEPROM (Electrically Erasable Programmable Read-Only Memory), PROM (Programmable Read-Only Memory), a magnetic memory, a magnetic disk, or an optical disk.

An embodiment of the disclosure may be implemented or supported by one or more computer programs, and the computer programs may be formed from computer-readable program code and may be included in a computer-readable medium. In the disclosure, the terms "application" and "program" may refer to one or more computer programs, software components, instruction sets, procedures, functions, objects, classes, instances, related data, or a portion thereof suitable for implementation in computer-readable program code. The "computer readable program code" may include various types of computer code including source code, object code, and executable code. The "computer-readable medium" may include various types of mediums accessed by a computer, such as read only memories (ROMs), random access memories (RAMs), hard disk drives (HDDs), compact disks (CDs), digital video disks (DVDs), or various types of memories.

Also, a machine-readable storage medium may be provided in the form of a non-transitory storage medium. Here, the 'non-transitory storage medium' may be a tangible device and may exclude wired, wireless, optical, or other communication links for transmitting temporary electrical or other signals. Moreover, the 'non-transitory storage medium' may not distinguish between a case where data is semipermanently stored in the storage medium and a case where data is temporarily stored therein. For example, the "non-transitory storage medium" may include a buffer in which data is temporarily stored. The computer-readable medium may be any available medium accessible by a computer and may include volatile or non-volatile medium and removable or non-removable medium. The computer-readable medium may include a medium in which data may be permanently stored and a medium in which data may be stored and may be overwritten later, such as a rewritable optical disk or an erasable memory device.

According to an embodiment of the disclosure, the method according to various embodiments of the disclosure may be included and provided in a computer program product. The computer program product may be traded as a product between a seller and a buyer. The computer program product may be distributed in the form of a machine-readable storage medium (e.g., a compact disk read only memory (CD-ROM)) or may be distributed (e.g., downloaded or uploaded) online through an application store or directly between two user devices. In the case of online distribution, at least a portion of the computer program product (e.g., a downloadable app) may be at least temporarily stored or temporarily generated in a machine-readable storage medium such as a manufacturer's server, a server of an application store, or a memory of a relay server.

The foregoing is illustrative of embodiments of the disclosure, and those of ordinary skill in the art will readily understand that various modifications may be made therein without materially departing from the spirit or features of the disclosure. Therefore, it is to be understood that the embodiments described above should be considered in a descriptive sense only and not for purposes of limitation. For example, each component described as a single type may also be implemented in a distributed manner, and likewise, components described as being distributed may also be implemented in a combined form.

The scope of the disclosure is defined not by the above detailed description but by the following claims, and all modifications derived from the meaning and scope of the claims and equivalent concepts thereof should be construed as being included in the scope of the disclosure.

## Claims

1. A method, performed by an augmented reality (AR) device, of measuring a user's vision, the method comprising:
obtaining a background image comprising an image of at least one physical object by capturing a front region of the AR device by using an outward facing camera included in the AR device;
identifying an edge of the image of the at least one physical object in the background image;
determining a first region for measuring the user's vision on the background image based on the identified edge;
determining a second region corresponding to the determined first region on a display included in the AR device;
outputting a virtual object for measuring the user's vision to the determined second region;
obtaining a user input signal for vision measurement after outputting the virtual object; and
calculating a vision prescription value of a user based on the obtained user input signal.

2. The method of claim 1, wherein the identifying of the edge of the image of the at least one physical object in the background image includes determining, as the edge, a part whose intensity varies by a preset threshold value or more over adjacent pixels on the background image.

3. The method of claim 1, wherein the obtaining of the background image includes
obtaining a depth map of the background image by using a depth sensor included in the AR device; and
identifying a depth value and a shape of the at least one physical object included in the background image based on the obtained depth map.

4. The method of claim 3, wherein the determining of the first region for measuring the user's vision on the background image based on the identified edge includes determining the first region on the background image based on the depth value and the shape of the at least one physical object and the identified edge.

5. The method of claim 1, further comprising:
identifying a focal distance from a user's eye to the virtual object;
calculating a test vision compensation value based on the identified focal length; and
compensating for the calculated vision prescription value based on the test vision compensation value.

6. The method of claim 5, wherein the identifying of the focal distance from the user's eye to the virtual object includes
identifying a physical object corresponding to the first region corresponding to the second region where the virtual object is displayed; and
identifying a focal distance from the user's eye to the identified physical object by using at least one of a LIDAR, a depth sensor, or an eye tracking sensor included in the AR device.

7. The method of claim 5, wherein the calculating of the test vision compensation value includes calculating the test vision compensation value based on a reciprocal (1/D) of the identified focal distance (D).

8. The method of claim 1, further comprising:
identifying a color of the first region; and
determining a color of the virtual object for measuring the user's vision based on the identified color of the first region.

9. The method of claim 8, wherein the color of the virtual object for measuring the user's vision is determined to have a maximum contrast with the color of the first region corresponding thereto.

10. The method of claim 8, further comprising: lowering brightness of pixels included in the second region, except for pixels in which the virtual object for measuring the user's vision is output, on the display.

11. The method of claim 1, further comprising: determining at least one of sizes or a number of virtual objects for measuring the user's vision based on an area of the first region.

12. The method of claim 1, wherein the determining of the second region corresponding to the determined first region includes
determining the second region so that the virtual object for measuring the user's vision is displayed on the display to overlay on the certain first region, by using an object locking mechanism.

13. The method of claim 1, further comprising:
recognizing a gaze direction of the user; and
outputting a guide indicator to the display when it is determined that the recognized gaze direction of the user is not toward the virtual object.

14. The method of claim 1, further comprising: controlling a variable focus lens included in the AR device based on the calculated vision prescription value of the user.

15. An augmented reality (AR) device measuring a user's vision, the AR device comprising:
an outward facing camera configured to obtain a background image comprising an image of at least one physical object by capturing a front region;
an output unit configured to output a virtual object for measuring the user's vision;
a user input unit configured to obtain a user input signal for a vision measurement after outputting the virtual object;
a storage storing a program comprising one or more instructions; and
at least one processor configured to execute the one or more instructions stored in the storage to
identify an edge of the image of the at least one physical object in the obtained background image;
determine a first region for measuring the user's vision on the background image based on the identified edge;
determine a second region corresponding to the determined first region on a display included in the output unit;
output the virtual object to the determined second region; and
calculate a vision prescription value of a user based on the obtained user input signal.
